# EUROPEAN PATENT APPLICATION

(11) **EP 4 364 721 A1**
(43) Date of publication of application: **08.05.2024**
(21) Application number: 22831577.6
(22) Date of filing: 30.05.2022
(51) Int. Cl.: A61K 8/34, A61K 8/44, A61Q 5/12

(54) **AMINO ACID SCALP AND HAIR CARE COMPOSITION, SCALP AND HAIR CARE PRODUCT CONTAINING SAID COMPOSITION AND PREPARATION METHOD**

(30) Priority: 28.06.2021 CN 202110719457
(71) Applicant: Nanjing Huashi New Material Co., Ltd., Nanjing, Jiangsu 210000 (CN)
(72) Inventor: LIN, Xianting, Nanjing, Jiangsu 210000 (CN); WU, Jun, Nanjing, Jiangsu 210000 (CN); YANG, Jianzhong, Nanjing, Jiangsu 210000 (CN); SU, Guizhen, Nanjing, Jiangsu 210000 (CN)
(74) Representative: Renaudo, Adrien Hanouar
(86) International application number: PCT/CN2022/095902
(87) International publication number: WO 2023/273755

(57) **Abstract**

Disclosed is an amino acid-based scalp and hair care composition, characterized in that the composition comprises the following components by weight percentage: Long carbon chain fatty acyl amino acid-based surfactants 0.5-5%, higher aliphatic alcohols 0.5-15%, and the remainder being solvent. Also disclosed are a scalp and hair care product containing the composition and a preparation method thereof. Amino acid-based scalp and hair care composition of the present invention takes the long carbon chain fatty acyl amino acid-based anionic surfactants as the active ingredient. When applied to the scalp and hair care product, the composition can repair the hydrophobicity of hair surface through the long carbon chain, achieve the hair care effect, and reduce the irritation to the scalp.

## Description

### Technical Field

The invention belongs to the field of daily chemical product, which particularly relates to an amino acid-based scalp and hair care composition, a scalp and hair care product containing the composition and a preparation method.

### Background of the Invention

The hair is comprised of three parts including hair medulla, hair cortex and hair cuticle from inside to outside. The hair cuticle on the hair surface, in the shape of overlapped scales, is covered with naturally hydrophobic 18-MEA fatty acid protective film. When the hair suffers damage from physical, chemical, heating, sunlight, climate and other external factors, the 18-MEA on the surface is first destroyed, and then the hair surface is changed from hydrophobicity to hydrophilicity with more negative charges, thereby leading to raised and missing hair cuticles, split ends, hollow hair, and other problems, and giving the impression of "dull", "rough", and "dry" hair. At the same time, the increased interaction between the damaged and relatively hydrophilic hair and the skin, makes the hair feel less smooth. In addition, the increased interaction between the damaged and relatively hydrophilic hairs also makes the hair more prone to tangling and breaking. Therefore, repairing the hydrophobicity of hair surface is a crucial hair care technique. For example, the technical hair care ingredient commonly used in the hair care products (including hair conditioner, hair mask, and the like) is long-chain alkyl cationic surfactants. On the one hand, the positively charged hydrophilic groups on the cationic surfactants can effectively adhere to the hair by attracting the negative charges on the damaged hair surface; on the other hand, the long-chain alkyl groups of C16-22 can achieve a similar hydrophobic effect as 18-MEA, thereby achieving a repair effect on the damaged hair surface. In addition, long-chain alkyl cationic surfactants and higher fatty alcohols can form a bimolecular film layered gel network structure through emulsification technology, which can not only provide a smooth feeling for hair care product during the application and rinsing stages, but also effectively deliver various functional ingredients. However, compared to other commonly used anionic surfactants, ampholytic surfactants and non-ionic surfactants, cationic surfactants have the greatest irritation to the skin. Lengthening the carbon chain of alkyl portion can only alleviate the irritation to a certain extent. Therefore, the Cosmetic Act of the PRC stipulates the use limit of long-chain alkyl cationic surfactants, such as 22-carbon chain alkyl trimethyl ammonium chloride and 18-carbon chain alkyl trimethyl ammonium chloride commonly used in the hair conditioner. For this reason, the rinse type hair care products on the market so far have focused on the hair care effect. It is generally not recommended for consumers to contact the product with scalp or apply the product to scalp.

The present invention relates to the application of relatively mild long carbon chain fatty acyl amino acid-based anionic surfactants in hair care product, and further provides an amino acid-based scalp and hair care composition without cations.

### Summary of the Invention

To solve these problems, the present invention provides an amino acid-based scalp and hair care composition, a scalp and hair care product containing the composition and a preparation method. The composition in the present invention, when applied in the scalp and hair care product, can repair the hydrophobicity of hair surface through the long carbon chain and achieve the hair care effect, while also being gentle on the scalp.

The objective of the present invention and the solutions to its technical problems are achieved through the following technical proposal.

The present invention provides an amino acid-based scalp and hair care composition. The composition comprises the following components by weight percentage: Long carbon chain fatty acyl amino acid-based surfactants 0.5-5%, higher aliphatic alcohols 0.5-15%, and the remainder being solvent.

Preferably, the long carbon chain fatty acyl amino acid-based surfactants comprise one or more of the following items: N-fatty acyl glutamic acid and its salts, N-fatty acyl alanine and its salts, N-fatty acyl-N-methyl-β-alanine and its salts, N-fatty acyl sarcosine and its salts, N-fatty acyl glycine and its salts, N-fatty acyl lysine and its salts, N-fatty acyl serine and its salts, N-fatty acyl cystine and its salts, N-fatty acyl cysteine and its salts, N-fatty acyl threonine and its salts, N-fatty acyl-L-valine and its salts, and N-fatty acyl aspartic acid and its salts.

Preferably, the long carbon chain fatty acyl groups in the long carbon chain fatty acyl amino acid-based surfactants are saturated and unsaturated fatty acid groups, with a carbon chain length of more than 16 carbon atoms. More preferably, the length of carbon chain is 16-22 carbon atoms.

Preferably, the number of carbon atoms in the higher fatty alcohols is ≥16, including one or more of the following items: cetyl alcohol, stearyl alcohol, behenol alcohol, arachyl alcohol, and hexadecanol/octadecanol mixture.

Preferably, the solvent is selected from water and/or polyols.

The objective of the present invention and the solutions to its technical problems are further achieved through the following technical proposal.

The present invention further provides a scalp and hair care product. The scalp and hair care product comprises the amino acid-based scalp and hair care composition.

Preferably, the weight percentage of the amino acid-based scalp and hair care composition in the scalp and hair care product is 0.01-99.99%.

Preferably, the scalp and hair care product also comprises hair care oil, essence, chelating agent, preservative and PH regulator. The volumes of addition for hair care oil, essence, chelating agent, preservative and PH regulator are known to those skilled in the art. Generally, pH regulator regulates the product to the acidic to neutral range. Of course, those skilled in the art shall easily understand that, according to the actual use demands, the scalp and hair care product can be added with anti-dandruff, anti-pruritic, anti-aging, moisturizing and other functional components.

Preferably, the scalp and hair care product does not comprise cationic surfactants or/and cationic macromolecular polymers.

Preferably, the scalp and hair care product is selected from one or more of the following items: hair conditioner, hair cream, hair balm, hair ointment, hair essence, hair mask, hair dressing gel, scalp care conditioner, scalp care cream, scalp massage ointment, scalp massage cream, and scalp essence.

The objective of the present invention and the solutions to its technical problems are further achieved through the following technical proposal.

The present invention also provides a preparation method of scalp and hair care product. The method comprises the following steps:
Dissolve the chelating agent and pH regulator into water at room temperature, add long carbon chain fatty acyl amino acid-based surfactants and higher aliphatic alcohols after being heated to 80-85°C, have homogeneous emulsification for 10-15 minutes after complete dissolution, and then make cooling while stirring. After cooling to 50-55°C, add hair care oil, preservative and essence, and stir them until they are uniformly mixed.

With the above technical proposals, the present invention has at least the following advantages: Amino acid-based scalp and hair care composition of the present invention takes the long carbon chain fatty acyl amino acid-based anionic surfactants, higher aliphatic alcohols and solvent as the active ingredients. When applied to the scalp and hair care product, the composition can repair the hydrophobicity of hair surface through the long carbon chain, achieve the hair care effect, and reduce the irritation to the scalp.

The foregoing description is only an overview of the technical proposals of the present invention. In order to make the technical means of the present invention more clearly understood and implemented in accordance with the contents of the description, the following detailed description is given with reference to the preferred embodiments of the present invention.

### Detailed Description of the Preferred Embodiments

To make the technical means, the creation features, the achievement purposes and the effects of the present invention easy to understand, the technical proposals in the embodiments of the present invention will be clearly and completely described below with reference to the embodiments of the present invention, and it is obvious that the described embodiments are only a part but not all of the embodiments of the present invention. All other embodiments, which can be derived by those skilled in the art from the embodiments given herein without making any creative effort, shall fall within the protection scope of the present invention.

### Examples 1-18: Preparation of scalp and hair care products

Unless otherwise specified, the long carbon chain fatty acyl amino acid-based anionic surfactants stated in the examples are from Nanjing Huashi New Material Co., Ltd. The higher aliphatic alcohols are purchased from New Japan Chemical Co., Ltd. The remaining components are all general commercially available products.

The scalp and hair care product is prepared according to the components and contents (weight percentage) in Tables 1-3. The preparation method is as follows: Dissolve EDTA-2Na and pH regulator into water at room temperature, add long carbon chain fatty acyl amino acid-based surfactants and higher aliphatic alcohols after being heated to 80-85°C, have homogeneous emulsification for 10-15 minutes after complete dissolution, and then make cooling while stirring. After cooling to 50-55°C, add hair care oil, preservative and essence, and stir them until they are uniformly mixed.

**Table 1: Components and Contents of Scalp and Hair Care Products in Examples 1-6**

| | Component | Example 1 | Example 2 | Example 3 | Example 4 | Example 5 | Example 6 |
|---|---|---|---|---|---|---|---|
| Scalp and hair care composition | C-18 acyl sodium alanine | - | 1.6 | 0.5 | 0.8 | 2.5 | 5 |
| | C-22 acyl sodium alanine | 2.35 | - | 2 | - | - | - |
| | Cetyl alcohol | 2.26 | 3.58 | 7.5 | 2.8 | - | 9 |
| | Stearyl alcohol | 3.39 | - | - | 1.4 | 4.6 | 6 |
| | Behenol alcohol | - | 2. 81 | - | - | 6.9 | - |
| | Water | 88.87 | 87.28 | 84.07 | 91.67 | 80.67 | 75.57 |
| Hair care oil | Polydimethylsiloxane | 1 | - | - | 0.5 | 3 | 2 |
| | Squalane | 0.5 | 2.4 | 4 | 0.5 | - | - |
| Chelating agent | EDTA-2Na | 0.13 | 0.13 | 0.13 | 0.13 | 0.13 | 0.13 |
| Preservative | Benzyl alcohol | 0.4 | 0.4 | 0.4 | 0.4 | 0.4 | 0.4 |
| | Phenoxyethanol | 0.4 | 0.4 | 0.4 | 0.4 | 0.4 | 0.4 |
| Essence | Essence | 0.5 | 0.5 | 0.5 | 0.5 | 0.5 | 0.5 |
| pH regulator | Citric acid | 0.2 | 0.9 | 0.5 | 0.9 | 0.9 | 1.0 |

**Table 2: Components and Contents of Scalp and Hair Care Products in Examples 7-12**

| | Component | Example 7 | Example 8 | Example 9 | Example 10 | Example 11 | Example 12 |
|---|---|---|---|---|---|---|---|
| Scalp and hair care composition | C-18 acyl sodium glutamate | 1.1 | 4 | - | 2.8 | - | 2.4 |
| | C-22 acyl sodium glutamate | - | - | 3.7 | 1 | 1.9 | - |
| | Cetyl alcohol | - | 4 | 8.72 | 0.7 | 1.6 | 2.24 |
| | Stearyl alcohol | - | - | 0.58 | - | 2.4 | 3.36 |
| | Hexadecanol/octadec anol | 0.5 | 6 | - | 10.5 | - | - |
| | Water | 95.97 | 82.27 | 83.97 | 82.37 | 90.57 | 87.77 |
| Hair care oil | Polydimethylsiloxane | 0.5 | 2 | - | 1 | 1 | - |
| | Squalane | 0.2 | - | 1.5 | - | 1 | 2.5 |
| Chelating agent | EDTA-2Na | 0.13 | 0.13 | 0.13 | 0.13 | 0.13 | 0.13 |
| Preservative | Benzyl alcohol | 0.4 | 0.4 | 0.4 | 0.4 | 0.4 | 0.4 |
| | Phenoxyethanol | 0.4 | 0.4 | 0.4 | 0.4 | 0.4 | 0.4 |
| Essence | Essence | 0.5 | 0.5 | 0.5 | 0.5 | 0.5 | 0.5 |
| pH regulator | Citric acid | 0.3 | 0.3 | 0.1 | 0.2 | 0.1 | 0.3 |

**Table 3: Components and Contents of Scalp and Hair Care Products in Examples 13-18**

| | Component | Example 13 | Example 14 | Example 15 | Example 16 | Example 17 | Example 18 |
|---|---|---|---|---|---|---|---|
| Scalp and hair care composition | C-18 acyl sodium cystine | 3.9 | 1.2 | 0.5 | - | 3 | - |
| | C-18 acyl sodium cysteine | - | - | 1.05 | 4.1 | 0.6 | 4.6 |
| | Cetyl alcohol | 2.84 | - | - | 4.36 | 4.44 | 8.64 |
| | Stearyl alcohol | 4.26 | 1.12 | - | 6.54 | - | 5.76 |
| | Hexadecanol/octadecanol | | 1.68 | 4.45 | - | 2.96 | - |
| | Water | 85.42 | 93.47 | 91.47 | 81.92 | 86.12 | 78.42 |
| Hair care oil | Polydimethylsiloxane | - | 0.2 | - | 1.5 | - | 0.5 |
| | Squalane | 2 | 0.8 | 1 | - | 1.3 | 0.5 |
| Chelating agent | EDTA-2Na | 0.13 | 0.13 | 0.13 | 0.13 | 0.13 | 0.13 |
| Preservative | Benzyl alcohol | 0.4 | 0.4 | 0.4 | 0.4 | 0.4 | 0.4 |
| | Phenoxyethanol | 0.4 | 0.4 | 0.4 | 0.4 | 0.4 | 0.4 |
| Essence | Essence | 0.5 | 0.5 | 0.5 | 0.5 | 0.5 | 0.5 |
| pH regulator | Citric acid | 0.15 | 0.1 | 0.1 | 0.15 | 0.15 | 0.15 |

### Test example 1: Basic performance test on the scalp and hair care products

Test object: Scalp and hair care products prepared in examples 1-18

### Test method:

pH test: Use the pH tester METTLER TOLEDO to determine the sample at room temperature.

Viscosity test: Use the viscosity tester Brookfield DV II to test the sample at room temperature and 20 rpm rotating speed using No. 93 rotor.

Appearance test: Visually determine the state.

Test results: See Tables 4 and 5.

**Table 4: Components and Contents of Scalp and Hair Care Products in Examples 1-9**

| Performance | Example 1 | Example 2 | Example 3 | Example 4 | Example 5 | Example 6 | Example 7 | Example 8 | Example 9 |
|---|---|---|---|---|---|---|---|---|---|
| pH | 6.9 | 6.1 | 6.45 | 6.5 | 6.21 | 6.4 | 5.87 | 6.01 | 6.3 |
| Viscosity mPa.s | 4000 | 3800 | 7600 | 3100 | 12000 | 17500 | 1500 | 13400 | 10500 |
| Appearance | White cream | White cream | White cream | White cream | White paste | White paste | White emulsion | White paste | White paste |

**Table 5: Components and Contents of Scalp and Hair Care Products in Examples 10-18**

| Perform ance | Example 10 | Example 11 | Example 12 | Example 13 | Example 14 | Example 15 | Example 16 | Example 17 | Example 18 |
|---|---|---|---|---|---|---|---|---|---|
| pH | 5.96 | 6.12 | 6.2 | 6.25 | 6.18 | 6.8 | 6.55 | 6.4 | 6.3 |
| mPa.s Viscosit y mPa.s | 14000 | 3450 | 5100 | 8400 | 2700 | 4000 | 13900 | 9500 | 17000 |
| Appeara nce | White paste | White cream | White cream | White cream | White emulsion | White cream | White paste | White paste | White paste |

According to the results in Tables 4 and 5, examples 1-18 are all within the normal indicator ranges of the products used by consumers.

### Test example 2: Hair care performance test on the scalp and hair care products

Test object: Scalp and hair care products prepared in examples 1-18

Test method: Apply the test object on the hair, rinse with warm water at around 40°C, and brush the hair with a comb while rinsing. After rinsing is completed, let the hair stand still until natural drying. A comprehensive evaluation on the test object regarding hair care performance is carried out through evaluating the hair combing smoothness during rinsing and after drying.

Test results: See Table 6.

**Table 6: Hair Care Performance of Scalp and Hair Care Products in Examples 1-18**

| | Example 1 | Example 2 | Example 3 | Example 4 | Example 5 | Example 6 | Example 7 | Example 8 | Example 9 |
|---|---|---|---|---|---|---|---|---|---|
| Hair care perfor mance | +++ | ++ | ++ | + | +++ | +++ | + | +++ | + |

| | Example 10 | Example 11 | Example 12 | Example 13 | Example 14 | Example 15 | Example 16 | Example 17 | Example 18 |
|---|---|---|---|---|---|---|---|---|---|
| Hair care perfor mance | ++ | + | ++ | + | + | + | ++ | ++ | +++ |

| | | | | | | | | | |
|---|---|---|---|---|---|---|---|---|---|
| Note: + + +: Excellent performance; + +: Good performance; +: Average performance; : Slightly poor performance; --: Poor performance; ---: Very poor performance; | | | | | | | | | |

According to the results in Table 6, the scalp and hair care products in Examples 1-18 can achieve the hair care effect above average performance.

The above is only a preferred embodiment of the present invention, and is not intended to limit the scope of the present invention. Although the present invention has been disclosed in the above preferred embodiments, it is not intended to limit the present invention. Those skilled in the art can make some modifications or modifications to the equivalent embodiments by using the above-disclosed technical contents without departing from the technical scope of the present invention, but without departing from the technical solution of the present invention, according to the present invention. Any modification, equivalent change and modification of the above embodiments according to the technical substantials of the present invention are still within the scope of the technical solution of the present invention.

## Claims

1. An amino acid-based scalp and hair care composition, **characterized in that** the composition comprises the following components by weight percentage: Long carbon chain fatty acyl amino acid-based surfactants 0.5-5%, higher aliphatic alcohols 0.5-15%, and the remainder being solvent.

2. The amino acid-based scalp and hair care composition according to Claim 1, **characterized in that** the long carbon chain fatty acyl amino acid-based surfactants comprise one or more of the following items: N-fatty acyl glutamic acid and its salts, N-fatty acyl alanine and its salts, N-fatty acyl-N-methyl-β-alanine and its salts, N-fatty acyl sarcosine and its salts, N-fatty acyl glycine and its salts, N-fatty acyl lysine and its salts, N-fatty acyl serine and its salts, N-fatty acyl cystine and its salts, N-fatty acyl cysteine and its salts, N-fatty acyl threonine and its salts, N-fatty acyl-L-valine and its salts, and N-fatty acyl aspartic acid and its salts.

3. The amino acid-based scalp and hair care composition according to Claim 1, **characterized in that** the number of carbon atoms in the higher fatty alcohols is ≥16, including one or more of the following items: cetyl alcohol, stearyl alcohol, behenol alcohol, and arachyl alcohol, hexadecanol/octadecanol mixture.

4. The amino acid-based scalp and hair care composition according to Claim 1, **characterized in that** the solvent is selected from water and/or polyols.

5. A scalp and hair care product, **characterized in that** the product comprises an amino acid-based scalp and hair care composition as claimed in any of Claims 1 to 4.

6. The scalp and hair care product according to Claim 5, **characterized in that** the weight percentage of the amino acid-based scalp and hair care composition in the scalp and hair care product is 0.01-99.99%.

7. The scalp and hair care product according to Claim 5, **characterized in that** the scalp and hair care product further comprises hair care oil, essence, chelating agent, preservative and PH regulator.

8. The scalp and hair care product according to Claim 5, **characterized in that** the product does not comprise cationic surfactants or/and cationic macromolecular polymers.

9. The scalp and hair care product according to Claim 5, **characterized in that** the product is selected from one or more of the following items: hair conditioner, hair cream, hair balm, hair ointment, hair essence, hair mask, hair dressing gel, scalp care conditioner, scalp care cream, scalp massage ointment, scalp massage cream, and scalp essence.

10. A preparation method of scalp and hair care product, **characterized in that** the method comprises the following steps:
dissolve the chelating agent and pH regulator into water at room temperature, add long carbon chain fatty acyl amino acid-based surfactants and higher aliphatic alcohols after being heated to 80-85°C, have homogeneous emulsification for 10-15 minutes after complete dissolution, and then make cooling while stirring. After cooling to 50-55°C, add hair care oil, preservative and essence, and stir them until they are uniformly mixed.
